# EUROPEAN PATENT SPECIFICATION

(11) **EP 1 035 859 B1**
(45) Date of publication and mention of the grant of the patent: **04.02.2004**
(21) Application number: 98959890.9
(22) Date of filing: 30.11.1998
(51) Int. Cl.: A61K 35/78, A61K 31/35

(54) **THE USE OF PHOSPHOLIPID COMPLEXES OF EXTRACTS OF VITIS VINIFERA AS ANTI-ATHEROSCLEROTIC AGENTS**
VERWENDUNG VON VITIS VINIFERA EXTRAKTEN PHOSPHOLIPID KOMPLEXEN ALS ANTIATHEROSCLEROTISCHE MITTELN
UTILISATION DE COMPLEXES PHOSPHOLIPIDIQUES D'EXTRAITS DE VITIS VINIFERA COMME AGENTS ANTI-ATHEROSCLEREUX

(30) Priority: 04.12.1997 IT MI972690
(43) Date of publication of application: 20.09.2000
(73) Proprietor: INDENA S.p.A., 20139 Milano (IT)
(72) Inventor: MORAZZONI, Paolo, I-20139 Milano (IT); BOMBARDELLI, Ezio, I-20141 Milano (IT)
(74) Representative: Minoja, Fabrizio, Dr.
(86) International application number: PCT/EP1998/007662
(87) International publication number: WO 1999/029331

(56) References cited:
- EP-A- 0 713 706
- US-A- 4 963 527
- K. TEBIB ET AL.: "ANTIOXYDANT EFFECTS OF DIETARY POLYMERIC GRAPE SEED TANNINS IN TISSUES OF RATS FED A HIGH CHOLESTEROL-VITAMIN E -DEFICIENT DIET" FOOD CHEMISTRY, vol. 59, no. 1, 1997, pages 135-141, XP002098281
- E. BOMBARDELLI ET AL.: "BIOLOGICAL ACTIVITY OF PROCYANIDINS FROM VITIS VINIFERA L." BIOFACTORS, vol. 6, no. 4, 1997, pages 429-431, XP002098282

## Description

The present invention relates to the use of phospholipid complexes of oligomeric proanthocyanidins containing from 2 to 7 catechin units extracted from *Vitis vinifera* for the preparation of medicaments for the treatment and the prophylaxis of atherosclerosis, and of miocardiac and cerebral infarctions.

The extracts of *Vitis vinifera* are already known and used in the therapy of cardiovascular disorders connected with venous insufficiency, in the treatment of impaired conditions of capillary permeability and resistance and in cicatrization. These extracts, which can be obtained from the seeds of the plant as described in GB-A-1,541,469 or in FR-A-2,092,743, are a mixture of polyphenols such as epicatechin and its polymerization products, in part esterified at the C-3 hydroxyl of the monomer with gallic acid. The phospholipid complexes of the extracts of *Vitis vinifera* are described in US-4,963,527 and are at present commercially available under the trade mark Leucoselect.

It has now surprisingly been found that the complexes prepared according to US-4,963,527 exert, when administered systemically, preferably by the oral route, a marked anti-atherosclerotic activity both in animals and in humans.

More specifically, it has been found that the phospholipid complexes of oligomeric proanthocyanidins containing from 2 to 7 catechin units extracted from *Vitis vinifera*, prevent or reduce the formation of atherosclerotic plaques in a dose-depending relationship.

Such an activity was evidenced in rabbits fed with hypercholesterolemic diet, so as to induce atherosclerotic lesions similar to the human ones at the vascular level, particularly at the aortic arch, ventral aorta, carotids and cerebral vessels. In said model, the above mentioned phospholipid complexes change the macro and microscopical vascular condition reducing, compared with untreated animals, both the number of atheromatous plaques and their severity, with a surprising vascular-tissular benefit. In another atherosclerosis model, with the purpose of cerebral protection, wherein the vasal lumen of rabbit internal carotid had been surgically reduced, while administering a hypercholesterolemic diet rich in saturated fats, a decrease in carotid obstruction, a reduction of vessel walls thickness and an increased survival of the animals were evidenced. Furthermore, in atherosclerotic patients, a reduction in carotid obstruction due to atheromatous plaques and an improved carotid flow, evaluated by Doppler ultrasonography, were observed.

The phospholipid complexes of the proanthocyanidins extracted from *Vitis vinifera* can be used in suitable oral formulations, such as tablets, soft- or hard-gelatin capsules, at dosages ranging from 50 to 500 mg two-three times a day, depending on the severity of the disease to treat. The preparation of the pharmaceutical formulations can be carried out according to conventional techniques and excipients.

The following examples illustrate the invention in greater detail.

### Example 1

3 groups of 8 New Zealand rabbits each, were treated as follows:
Group 1): Control, normal diet
Group 2): Hypercholesterolemic diet (0.2% cholesterol)
Group 3): Hypercholesterolemic diet + phospholipid complex of extracts of *Vitis vinifera* (0.2% cholesterol + 2% Leucoselect^{(R)}).

After 8 weeks, during which cholesterol, LDL/VLDL, HDL and triglycerids levels were measured, the animals were killed.

The number, the size and the distribution of the atherosclerotic lesions on thoracic and abdominal aorta were evaluated.

Aorta strips were fixed and stained with Sudan IV to visualize the lesions and to evaluate the vasal cholesterol and the content in oxidized cholesterol by gaschromatography.

The results reported in the following table prove that the treatment with phospholipid complexes of extracts of *Vitis vinifera* decreases in a statistically significant way the atherosclerotic lesions induced by hypercholesterolemic diet.

**Table**

| Treatment | area percent of the lesion |
|---|---|
| Group 1 | 1% |
| Group 2 | 27% |
| Group 3 | 5%* |

| | |
|---|---|
| *p<0.01 compared with group 2 | |

### Example 2

| Capsules containing 500 mg of phospholipid complex of extracts of *Vitis vinifera* | |
|---|---|
| Composition: | |
| Complex of extract of *Vitis vinifera* | |
| with soy phosphatidylcholine | 200 mg |
| Lactose | 57 mg |
| Modified starch | 40 mg |
| Magnesium stearate | 3,0 mg |

### Example 3

| Gastro-resistant tablets | |
|---|---|
| Complex of extract of *Vitis vinifera* | |
| with soy phosphatidylcholine | 250 mg |
| Microcrystalline cellulose | 118 mg |
| Precipitated silica | 3 mg |
| Magnesium stearate | 4 mg |
| Methacrylic acid anionic polymer and esters thereof | 12 mg |
| Talc | 8 mg |
| Magnesium carbonate | 8.mg |
| Maize star | 5 mg |
| Gum arabic | 159 mg |

### Example 4

| Soft-gelatin capsules | |
|---|---|
| Complex of extract of *Vitis vinifera* | |
| with soy phosphatidylcholine | 266 mg |
| Peanut oil | 209 mg |
| Partially hydrogenated vegetable oils | 100 mg |
| Soy lecithin | 5 mg |

## Claims

1. The use of phospholipid complexes of oligomeric proanthocyanidins containing from 2 to 7 catechin units extracted from *Vitis vinifera* for the preparation of medicaments for the therapeutical and prophylactic treatment of atherosclerosis and of miocardiac and cerebral infarctions.

2. The use as claimed in claim 1, wherein the unitary dosage of the medicament ranges from 50 to 500 mg of active ingredient.

3. The use as claimed in claim 1 or 2, wherein the medicament is administrable orally.

4. The use as claimed in claim 3, wherein the medicament is in the form of soft- or hard- gelatin capsules or of tablets.

## Patentansprüche

1. Verwendung von Phospholipidkomplexen von oligomeren Proanthocyanidinen, die 2 bis 7 Catechineinheiten enthalten, extrahiert aus *Vitis vinifera*, zur Herstellung von Arzneimitteln für die therapeutische und prophylaktische Behandlung von Atherosklerose und von Herz- und Hirninfarkten.

2. Verwendung nach Anspruch 1, wobei die Dosierungseinheit des Arzneimittels im Bereich von 50 bis 500 mg Wirkstoff liegt.

3. Verwendung nach Anspruch 1 oder 2, wobei das Arzneimittel oral verabreicht werden kann.

4. Verwendung nach Anspruch 3, wobei das Arzneimittel in Form von Weich- oder Hartgelatinekapseln oder Tabletten vorliegt.

## Revendications

1. Utilisation de complexes phospholipidiques de proanthocyanidines oligomères contenant de 2 à 7 unités catéchine, extraites de *Vitis vinifera* pour la préparation de médicaments pour le traitement thérapeutique et prophylactique de l'athérosclérose et des infarctus du myocarde et cérébral.

2. Utilisation telle que revendiquée dans la revendication 1, pour laquelle le dosage unitaire du médicament se situe dans la plage allant de 50 à 500 mg d'ingrédient actif.

3. Utilisation telle que revendiquée dans la revendication 1 ou 2, pour laquelle le médicament est administrable par voie orale.

4. Utilisation telle que revendiquée dans la revendication 3, pour laquelle le médicament est sous la forme de capsules de gélatine molle ou dure ou de comprimés.
